(19) Europäisches Patentamt
European Patent Office
Office européen des brevets

(11) **EP 3 605 058 A1**

(12) **EUROPEAN PATENT APPLICATION**

(43) Date of publication:
**05.02.2020 Bulletin 2020/06**

(51) Int Cl.:
**G01N 11/00** (2006.01)  **G01N 25/50** (2006.01)

(21) Application number: **18186281.4**

(22) Date of filing: **30.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SABIC Global Technologies B.V.**
**4612 PX Bergen op Zoom (NL)**

(72) Inventors:
• **CHELLAMUTHU, Manojkumar**
**4612 PX Bergen op Zoom (NL)**

• **ASTHAN, Himanshu**
**4612 PX Bergen op Zoom (NL)**
• **ANDERSON, Zachary**
**4612 PX Bergen op Zoom (NL)**
• **SUR, Samrat**
**Boston, MA 02108 (US)**
• **ROTHSTEIN, Jonathan**
**Boston, MA 02108 (US)**

(74) Representative: **Lang, Johannes**
**Bardehle Pagenberg Partnerschaft mbB**
**Patentanwälte, Rechtsanwälte**
**Prinzregentenplatz 7**
**81675 München (DE)**

(54) **HIGH TEMPERATURE EXTENSIONAL RHEOLOGY MEASUREMENTS TO PREDICT THE FLAMMABILITY RELATED PROPERTY OF A MATERIAL**

(57) This disclosure describes a device, system, and method for using extensional rheology measurements to predict a flammability related property of a material, such as a flame retardant polycarbonate resin. For example, a method of generating a prediction associated with a flammability related property of a material includes determining a set of data values based on image data corresponding to a sample of the material under test. The set of data values are associated with a characteristic of the sample. The method also includes determining, based on the set of data values, an extensional viscosity value associated with the material, and generating the prediction based on a result of a comparison between the extensional viscosity and a threshold. In some implementation, the flammability related property includes an anti-drip property.

FIG. 1

**Description**

**FIELD OF INVENTION**

The present disclosure relates generally to predictions of a flammability related property of a material, and, but not by way of limitation, to methods, devices, and systems for predicting a flammability related property of a material, such as an anti-drip property.

**BACKGROUND**

Plastic materials are widely used in a variety of devices and appliances that include electronic equipment. In such implementations, the plastic materials need withstand heat energy and failure, including fire, of the electronic equipment. For example, plastic materials incorporated into devices and appliances need to exhibit heat resistance, flame retardant properties, and anti-drip properties. To incorporate such properties in plastic materials, one or more flame retardant additives are typically added to a polymer.
A plastic material is rated according to one or more flammability standards which can determines the material's tendency to either extinguish or spread the flame once the specimen has been ignited. An example of such as standard is UL 94, the Standard for Safety of Flammability of Plastic Materials for Parts in Devices and Appliances testing, is a plastics flammability standard released by Underwriters Laboratories (UL) of the United States. UL-94 is harmonized with IEC 60707, 60695-11-1-, and 6-695-11-2-, and ISO 9772 and 9773. To illustrate, a plastic material may have a rating based on one or more standards that are in effect as of June 1, 2018. Referring to UL 94 Flammability Standard, testing is performed on a material to determine a classification (e.g., a rating), such as UL 94 HB, V-2, V-1, V-0, 5VB, 5VA. Testing a plastic material for a standard-based rating can be time consuming and expensive.
When developing a plastic material that includes FR additives, one or more conventional techniques can be used to determine investigate the influence of FR additives. For example, Thermal Gravimetric Analysis, Mass Cone calorimetry and Pyrolysis-combustion flow calorimeter are common measurements to investigate the influence of FR additives in decomposition mechanisms and heat release. Such techniques can provide insight on some aspects of the flammability nature of a polymer that includes an FR additive. However, the conventional techniques do not provide details on what kind of rheological changes or decomposition mechanism (e.g., branching, crosslinking or chain scission via thermal degradation) may reduce or prevent drips. Stated in another manner, the conventional techniques do not provide details on the flow properties (e.g., the rheological changes occur during flame spread) of a polymer that includes an FR additive.

**SUMMARY**

The present disclosure describes predictions of a flammability related property (e.g., an anti-drip property) of a material, and methods, devices, and systems to predict a flammability related property of a material based on extensional rheology measurements. For example, the material may include a polymer that includes one or more FR additives. To illustrate, the material may include a flame retardant polycarbonate resins. Additionally, or alternatively, the material may include an ultra-low halogen additive or a halogen additive crosslinkable material. According to aspects of the present disclosure, to generate a prediction associated with a flammability related property of a material, a sample of the material may undergo an extensional rheology test, such as a high temperature extensional rheology test, in which the sample is heated to a temperature and stretched. For example, the sample may be heated to a temperature above a glass transition temperature ($T_g$) of the material and/or a polymer (e.g., polycarbonate) included in the material. In some implementations, the sample is heated to a temperature within a range (e.g., an inclusive or exclusive range) of 180-200 degrees Celsius (°C) above $T_g$. Image data (e.g., one or more images or video) may be generated that capture the sample during the extensional rheology test, and the image data may be processed to determine data values of a characteristic (e.g., a diameter decay) of the sample based on the extensional rheology test.
Based on the data values, an extensional viscosity value associated with the material may be determined and compared to a threshold. A prediction associated with a flammability related property, such as an anti-drip property, of the material may be determined based on a result of the comparison. For example, extensional viscosity of a material at one or more temperatures has been identified as being relevant to flame drips (e.g., the one or more temperatures may be correlated to a flame drip property). To illustrate, it is believed that an extensional viscosity, such as an average extensional viscosity within a temperature range indicates/predicts an anti-drip property during a standard-based tests, such as UL 94 testing. As an illustrative, non-limiting example, a material having an average extensional viscosity greater than or equal to 35,000 Pa.s (determined based on extensional rheology testing in the range from $T_g$ + 180 °C to $T_g$ + 220 °C), may reduce or avoid drips in UL 94 V testing. In a particular implementation, based on the extensional viscosity being greater than or equal to a threshold associated with a value of 35,000 pascal seconds (Pa.s), the material may be predicted to have a UL94 V0 rating. In some implementations, multiple samples of the material may each undergo an extensional rheology test at different temperatures to generate corresponding sets of data values associated with a diameter decay of the material (at the different temperatures). Each set of data values may be used to determine a corresponding extensional viscosity, and the extensional viscosity may be combined (e.g., averaged), and the combined extensional viscosity value may be compared to the threshold to generate the prediction.

Thus, the present disclosure describes techniques to predict a flammability related property (e.g., an anti-drip property) of a material. As an illustrative, non-limiting example, an anti-drip property of a FR polycarbonate resin may be predicted based on one or more extensional rheometer tests. The techniques of the present disclosure may use an extensional rheometer to advantageously mimic the process of dripping, similar to how a polymer would form a drop and drip during an actual fire, to generate an extensional viscosity value of a material at one or more temperatures. To illustrate, the present techniques have been used to correlate extensional viscosity to temperatures relevant to flame drips, thereby enabling predictions of a flammability related property (e.g., an anti-drip property) of a material. For example, the techniques described herein may be used to correlate an effect of a particular FR additive on a range of temperatures to identify a viscosity value and/or a range of temperatures for use in predicting a reduction or prevention of drips. Prior to the techniques described herein, such predictions have been unavailable using conventional techniques, such as Thermal Gravimetric Analysis, Mass Cone calorimetry and Pyrolysis-combustion flow calorimeter, that investigate the influence of FR additives. Accordingly, the present techniques beneficially enables an effect of a particular FR additive of a material to be determined/predicted as it relates to a flammability related property (e.g., a standard rating) of a material. The prediction of the flammability related property (e.g., the standard rating) may be determined independent of and/or without performance of the standard-based test, such as the UL 94 standard test. Accordingly, the present techniques enable a material formulation (e.g., an FR formulation of a polymer and one or more FR additives) to be optimized for a target or desired FR rating, such as a particular UL 94 rating. Additionally, the present techniques enable and promote development of new materials, such as materials that include an ultra-low a halogen additive or a halogen free additive (e.g., KSS salt), with flammability properties for a target or desired FR rating. Such optimization and/or development may be achieved with greater precision, in a shorter amount of time, at a reduced cost as compared to conventional techniques and/or testing to determine the flammability related property (e.g., the standard rating) of a material.

Some embodiments of the present systems comprise: an electronic device comprising a processor and a memory coupled to the processor, the memory storing instructions that, when executed by the processor, cause the processor to: determine a set of data values based on image data corresponding to a test of a sample of a material performed at an extensional rheometer, the image data associated with a time period and a temperature, the set of data values associated with a characteristic of the sample during the time period; determine, based on the set of data values, an extensional viscosity value associated with the material; and generate, based on a result of a comparison between the extensional viscosity and a threshold, a notification associated with a prediction of a flammability rating. In some such embodiments, the material comprises a flame retardant polycarbonate resin; the threshold corresponds to a value of 35,000 pascal seconds (Pa.s); or the flammability rating is associated with a UL 94 Flammability Standard. Additionally, or alternatively, the control device is further configured to: receive the first image data from a camera; and receive temperature data indicating the temperature value.

In some of the foregoing embodiments of the present systems, the flammability rating is associated with a UL 94 Flammability Standard; the prediction indicates a UL 94 V0 rating is achievable for the material based on an average extensional viscosity value being greater than or equal to 35,000 pascal seconds (Pa.s) within a range of 180-200 degrees Celsius (°C) above the glass transition temperature ($T_g$) of the material; and/or the prediction indicates a UL 94 V2 rating is achievable for the material based on an average extensional viscosity value being greater than or equal to 10,000 pascal seconds (Pa.s) within a range of 180-200 degrees Celsius (°C) above the glass transition temperature ($T_g$) of the material. In some such embodiments, the flammability rating is associated with a UL 94 Flammability Standard; and/or the material includes an ultra-low halogen additive or a halogen additive crosslinkable material.

In some of the foregoing embodiments of the present systems, the present systems further comprise: the extensional rheometer coupled to the electronic device, the extensional rheometer comprising: a housing defining a chamber, the housing having a window incorporated therein; a first coupler and a second coupler positioned within the chamber, the first and second coupler configured to hold a sample, the first coupler movable with respect to the second coupler; and a heat element positioned within the chamber. In some such embodiments, the present systems further comprise an image capture device coupled to the electronic device and positioned to have a field of view through the window, the camera configured to generate the image data.

Some embodiments of the present methods (e.g., of generating a prediction associated with a flammability related property of a material) comprise: determining, at a processor of an electronic device, a first set of data values based on first image data corresponding to a first sample of the material under test, the first image data associated with a first time period, the first set of data values associated with a characteristic of the first sample during the first time period; determining, based on the first set of data values, an extensional viscosity value associated with the material;

and generating, based on a result of a comparison between the extensional viscosity and a first threshold, a notification that indicates the prediction associated with the flammability related property of the material. In some such embodiments, an initial diameter of the first sample under test is greater than or equal to 1.5 mm and/or less than or equal to 3 mm, the prediction indicates the flammability rating is associated with a UL 94 Flammability Standard, an anti-drip property in the material, or both, and optionally, the material comprises a flame retardant polycarbonate resin.

In some of the foregoing embodiments of the present methods, the present methods further comprise: providing a gas into the chamber, wherein, optionally, the gas comprises nitrogen; adjusting a temperature within the chamber to a first temperature; and receiving the image data from a camera. In some such embodiments, the present methods further comprise, after the first sample is positioned between a first coupled and a second coupler within a chamber of a extensional rheometer: maintaining the first temperature within the chamber; and initiating movement of the first coupler and the second coupler; wherein, optionally, the first temperature is greater than a glass transition temperature (Tg) of the material.

In some of the foregoing embodiments of the present methods, determining the first set of data values based on the first image data comprises: performing edge detection on a first image based on the first image data, the first image corresponding to a first time during the first time period; determining a first data value of the first set of data values, wherein the first data value is associated with a first diameter of the first sample at the first time; performing edge detection on a second image based on the first image data, the second image corresponding to a second time during the first time period; and determining a second data value of the first set of data values, wherein the second data value is associated with a second diameter of the first sample at the second time. Additionally, or alternatively, in some embodiments, determining the extensional viscosity value comprises: performing spline interpolation on the first set of data to generate a first interpolated data set; and performing a differential operation on the first interpolated data set.

In some of the foregoing embodiments of the present methods, the present methods further comprise: prior to performing the comparison between the extensional viscosity and the first threshold, performing a comparison between the extensional viscosity and a second threshold; determining that the extensional viscosity is less than or equal to the second threshold, and wherein, optionally, the first threshold corresponds to a value of 35,000 pascal seconds (Pa.s). Additionally, or alternatively, each of the first temperature and the second temperature is greater than a glass transition temperature (Tg) of the material, and wherein the first temperature is different from the second temperature; and optionally, each of the first temperature and the second temperature is within a range of 180-200 degrees Celsius (°C) above the glass transition temperature (Tg) of the material.

In some of the foregoing embodiments of the present methods, the present methods further comprise: determining, at the processor, a second set of data values based on second image data corresponding to a second sample of the material, the second image data associated with a second time period, the second set of data values associated with a characteristic of the second sample during the second time period, wherein the extensional viscosity value is further determined based on the second set of data values, and wherein, optionally, the material includes an ultra-low halogen additive or a halogen additive. In some such embodiments, the material comprises: 100 grade PCP in a weight percentage of 18.3-18.9 and KSS in a weight percentage of 0.02-0.55, and, optionally, potassium perfluorobutane sulfonate in a weight percentage of 0.03-0.05; or PCP 1300 in a weight percentage of 57.6-58.2 and KSS in a weight percentage of 0.02-0.55, and, optionally, potassium perfluorobutane sulfonate in a weight percentage of 0.03-0.05.

As used herein, various terminology is for the purpose of describing particular implementations only and is not intended to be limiting of implementations. For example, as used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "or" is used to refer to a nonexclusive "or" unless otherwise indicated.

The term "about" as used herein can allow for a degree of variability in a value or range, for example, within 10%, within 5%, or within 1% of a stated value or of a stated limit of a range, and includes the exact stated value or range. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the term "substantially" may be substituted with "within [a percentage] of" what is specified, where the percentage includes .1, 1, or 5 percent; and the term "approximately" may be substituted with "within 10 percent of" what is specified. The phrase "and/or" means and or. To illustrate, A, B, and/or C includes: A alone, B alone, C alone, a combination of A and B, a combination of A and C, a combination of B and C, or a combination of A, B, and C. In other words, "and/or" operates as an inclusive or. The phrase "at least one of A and B" has the same meaning as "A, B, or A and B."

Throughout this document, values expressed in a range format should be interpreted in a flexible manner to include not only the numerical values explicitly recited as the limits of the range, but also to include all the individual numerical values or sub-ranges encompassed within that range as if each numerical value and sub-range is explicitly recited. For example, a range of "about 0.1 % to about 5%" or "about 0.1 % to 5%" should be interpreted to include not just about 0.1 % to about 5%, but also the individual values (e.g., 1 %, 2%, 3%, and 4%) and the sub-ranges (e.g., 0.1 % to 0.5%, 1.1 % to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise.

The terms "comprise" (and any form of comprise, such as "comprises" and "comprising"), "have" (and any form of have, such as "has" and "having"), and "include" (and any form of include, such as "includes" and "including"). As a result, an apparatus that "comprises," "has," or "includes" one or more elements possesses those one or more elements, but is not limited to possessing only those one or more elements. Likewise, a method that "comprises," "has," or "includes" one or more steps possesses those one or more steps, but is not limited to possessing only those one or more steps.

Any embodiment of any of the systems, methods, and article of manufacture can consist of or consist essentially of - rather than comprise/have/include - any of the described steps, elements, and/or features. Thus, in any of the claims, the term "consisting of' or "consisting essentially of' can be substituted for any of the open-ended linking verbs recited above, in order to change the scope of a given claim from what it would otherwise be using the open-ended linking verb. Additionally, it will be understood that the term "wherein" may be used interchangeably with "where."

Further, a device or system that is configured in a certain way is configured in at least that way, but it can also be configured in other ways than those specifically described. The feature or features of one embodiment may be applied to other embodiments, even though not described or illustrated, unless expressly prohibited by this disclosure or the nature of the embodiments. In the methods described herein, the acts can be carried out in any order, except when a temporal or operational sequence is explicitly recited. Furthermore, certain acts can be carried out concurrently even if described as being carried out sequentially. In addition, it is to be understood that the phraseology or terminology employed herein, and not otherwise defined, is for the purpose of description only and not of limitation. Any use of section headings is intended to aid reading of the document and is not to be interpreted as limiting; information that is relevant to a section heading may occur within or outside of that particular section.

Some details associated with the embodiments are described above, and others are described below. Other implementations, advantages, and features of the present disclosure will become apparent after review of the entire application, including the following sections: Brief Description of the Drawings, Detailed Description, and the Claims.

## BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiment depicted in the figures. Views identified as schematics are not drawn to scale.

FIG. 1 is a diagram that illustrates an example of a system to test a material to generate a prediction associated with a flammability related property of the material.

FIG. 2 is a flowchart illustrating a first example of a method of generating a prediction associated with a flammability related property of a material.

FIG. 3 is a flowchart illustrating a second example of a method generating a prediction associated with a flammability related property of a material.

FIG. 4 is a graph to illustrate test results of a first material to generate a prediction associated with a flammability related property of the first material.

FIG. 5 is a graph to illustrate test results of a second material to generate a prediction associated with a flammability related property of the second material.

FIG. 6 is a graph to illustrate test results of a third material to generate a prediction associated with a flammability related property of the third material.

FIG. 7 is a graph to illustrate test results of a fourth material to generate a prediction associated with a flammability related property of the fourth material.

FIG. 8 is a graph to illustrate test results of a fifth material to generate a prediction associated with a flammability related property of the fifth material.

FIG. 9 is a graph to illustrate test results of a sixth material to generate a prediction associated with a flammability related property of the sixth material.

FIG. 10 depicts graphs associated with the graphs and materials of FIGS. 4-9.

**DETAILED DESCRIPTION OF ILLUSTRATIVE EMBODIMENTS**

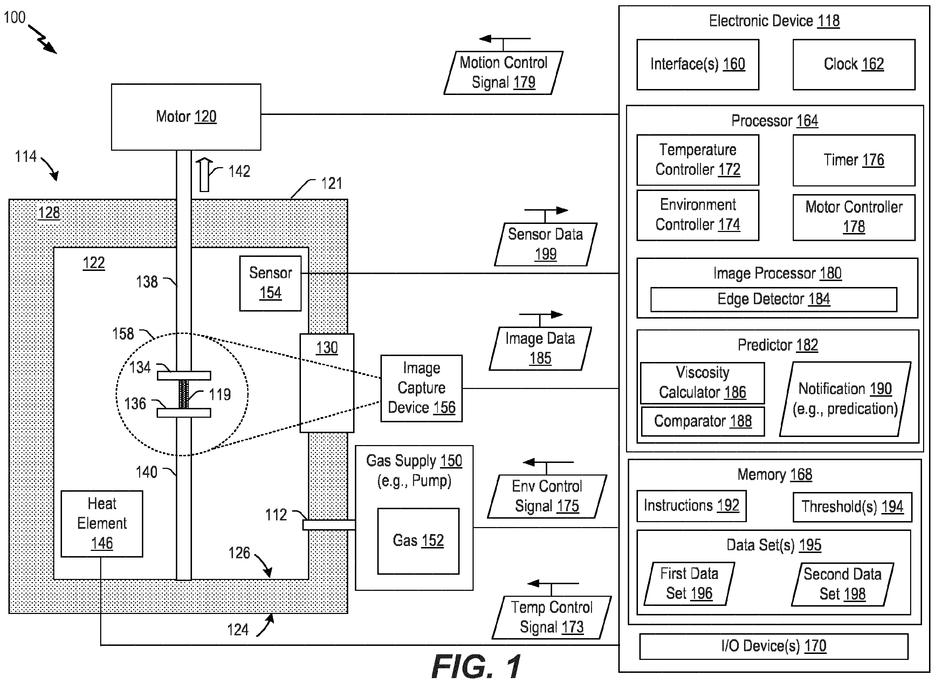

Referring to FIG. 1, a system 100 configured to test a material to generate a prediction associated with a flammability related property of the material, is shown. The system 100 includes an extensional rheometer 114 and an electronic device 118. As shown, extensional rheometer 114 is coupled electronic device 118, such as via one or more wired and/or wireless control lines. Although system 100 is described extensional rheometer 114 being coupled to electronic device 118, in other implementations, electronic device 118 may be separate and/or distinct from extensional rheometer 114. In such implementation, information or data may be exchanged between extensional rheometer 114 and electronic device 118 via an intermediate device (e.g., a memory card or thumb drive) or by an operator of system 100. For example, one or more operations of extensional rheometer 114 that are described herein as being controlled or initiated by electronic device 118 may additionally, or alternatively, be controlled locally (e.g., manually) at the extensional rheometer 114, such as by an operator of system 100.

Extensional rheometer 114, such as a extensional rheometer (e.g., a capillary breakup extensional rheometer), is configured to heat and stretch a sample 119 to generate extensional rheology measurements. Rheometer 114 includes a housing 121 defining a chamber 122. Housing 121 includes a first surface 124 (e.g., an outer surface) and a second surface 126 (e.g., an inner surface). Housing 121 also include an insulation material 128. In some implementations, a thickness of insulation material 128 (e.g., insulator) is configured to maintain a temperature within chamber 122 of T = 400 °C without exceeding an outside surface 124 temperature of 35 °C. In a particular implementation, insulation material 128 includes a silica-based insulator having a thickness of 6.25 cm and a thermal conductivity of k = 0.024 W/mK. Rheometer 114 is configured such that a temperature within chamber 122 is able to reach a temperature of greater than or equal to 400 °C. In some implementations, housing 121 includes an inner and outer box fabricated from steel plates with insulation between the two boxes to reduce heat transfer out of chamber 122.

Housing 121 includes one or more windows, such as a representative window 130, coupled to and/or incorporated therein. For example, openings may be cut into/through housing 121 and/or insulating material 128. In some implementations, a glass (e.g., Pyrex glass) may be inserted into or cover such openings to enable a line of sight to at least a portion of chamber 122.

Rheometer 114 further includes a first coupler 134 and a second coupler 136 positioned within chamber 122. For example, couplers 134, 136 may include cylindrical plates. First and second couplers 134, 136 are configured to hold sample 119, as descried further herein. First coupler 134 is coupled to a first support structure 138 and second coupler 136 is coupled to a second support structure 140. First coupler 134 is configured to be movable with respect to second coupler 136. For example, rheometer 114 includes a motor 120, or other device, configured to move first coupler 134 (e.g., structure 138) in a direction of travel as indicated by arrow 142. It is noted that motor 120 is also configured to move first coupler 134 (e.g., structure 138) in an opposite direction of travel from that indicated by arrow 112. In some implementation, motor 120 may be configured to move first coupler 134 (e.g., first support structure 138) at a rate of up to 200 mm/s. As shown, second coupler 136 (e.g., structure 140) is coupled to housing 121. In other implementations, second coupler 136 (e.g., structure 140) may be coupled to another motor and may be configured to move with reference to the first coupler 134. In a particular implementation, motor 120 includes a linear motor and first support structure 138 includes a glass mica rod, which may reduce heat flow from chamber 122 and minimize a risk of heat damage to motor 120.

Rheometer 114 further includes a heat element 146, gas supply 150, and one or more sensors 154. As shown heat element 146 is positioned within chamber 122. Heat element 146 may include one or more resistors, as an illustrative, non-limiting example. To illustrate, in a particular implementation, heat element 146 includes multiple (e.g., three) 250 W resistance heaters. Gas supply 150 may include a pump, a valve, regulator, a vacuum, a combination thereof, or the like. In some implementations, gas supply 150 is configured to provide gas 152 into the chamber 122. For example, gas 152 may include nitrogen, oxygen, or a combination thereof. To illustrate, gas 152 may be provided into chamber 122 to create an inert or an oxygen rich environment. One or more sensors 154 may include a temperature sensor (configured to generate temperature data to be provided to temperature controller), an air quality sensor (configured to generate air quality data to be provided to environmental sensor), a pressure sensor (configured to generate pressure data to be provided to environmental sensor), a combination thereof, or the like, as illustrative, non-limiting examples. To illustrate, the temperature data may indicate a temperature value associated with chamber 122.

An image capture device 156 may be coupled to electronic device 118 and positioned to have a field of view 158 through window 130. Image capture device 156 is configured to generate image data and may include a camera, video camera, etc., as illustrative, non-limiting examples.

Electronic device 118 includes one or more interfaces 160, a clock 162, one or more processors (e.g., one or more controllers), such as a representative processor 164, a memory 168, and one or more input/output (I/O) devices 170. Interfaces 160 may include a network interface and/or a device interface configured to be communicatively coupled to one or more other devices, such as rheometer 114 (e.g., motor 120, heat element 146, gas supply 150, sensor 154) or image capture device 156. For example, interfaces 160 may include a transmitter, a receiver, or a combination thereof (e.g., a transceiver), and may enable wired communication, wireless communication, or a combination thereof.

Processor 164 includes a temperature controller 172, an environment controller 174, a timer 176, a motor controller 178, an image processor 180, and a predictor 182. Temperature controller 172 is configured to control (or regulate) a temperature within chamber 122. For example, temperature controller 172 (e.g., processor 164) may be configured to generate and/or communicate one or more temperature control signals 173 to heat element 146. In a particular implementation, temperature controller 172 includes a proportional-integral-derivative (PID) temperature controller with a temperature accuracy of +/- 2 °C. Environment controller 174 is configured to control (or regulate) an environment, such as an air quality and/or pressure, within chamber. For example, environment controller 174 may be configured to generate and/or communicate one or more environment control signals 175 to gas supply 150.

Timer 176 is configured to monitor and/or determine expiration of one or more time periods (e.g., durations). In some implementations, timer 176 may be configured to operate based on and/or in association with clock 162. Motor controller 178 is configured to control operation of motor 120, which may impact a distance between couplers 134, 136. For example, motor controller 178 may be configured to generate and/or communicate motion control signal 179.

Image processor 180 is configured to process image data, such as image data 185 received from image capture device 156. To illustrate, image processor 180 may include an edge detector 184 configured to detect one or more edge based on received image data. In some implementations, image processor 180 may also be configured to generate data, such as one or more data sets. For example, the image processor 180 may determine a distance between two edges. To illustrate, the distance between two edges may be associated with a diameter of sample 119.

Predictor 182 is configured to generate a prediction associated with a flammability related property of a material, such as a material of sample 119. For example, predictor 182 may be configured to perform one or more operations to determine the prediction and, based on the prediction, may generate a notification 190 that associated with (e.g., indicates) the prediction. Predictor 182 includes a viscosity calculator 186 and a comparator 188. To illustrate, viscosity calculator 186 may determine a viscosity value associated with a material (of sample 119). Comparator 188 is configured to compared data values and/or thresholds.

Although one or more components of processor 164 are described as being separate components, at in some implementations, one or more components of the processor 164 may be combined into a single component. For example, although temperature controller 172 and environment controller 174 are described as being separate, in other implementations, temperature controller 172 and environment controller 174 may be incorporated into a single controller. Additionally, or alternatively, one or more components of processor 164 may be separate from (e.g., not included in) processor 164. To illustrate, image processor 180 may be separate and distinct from processor 164.

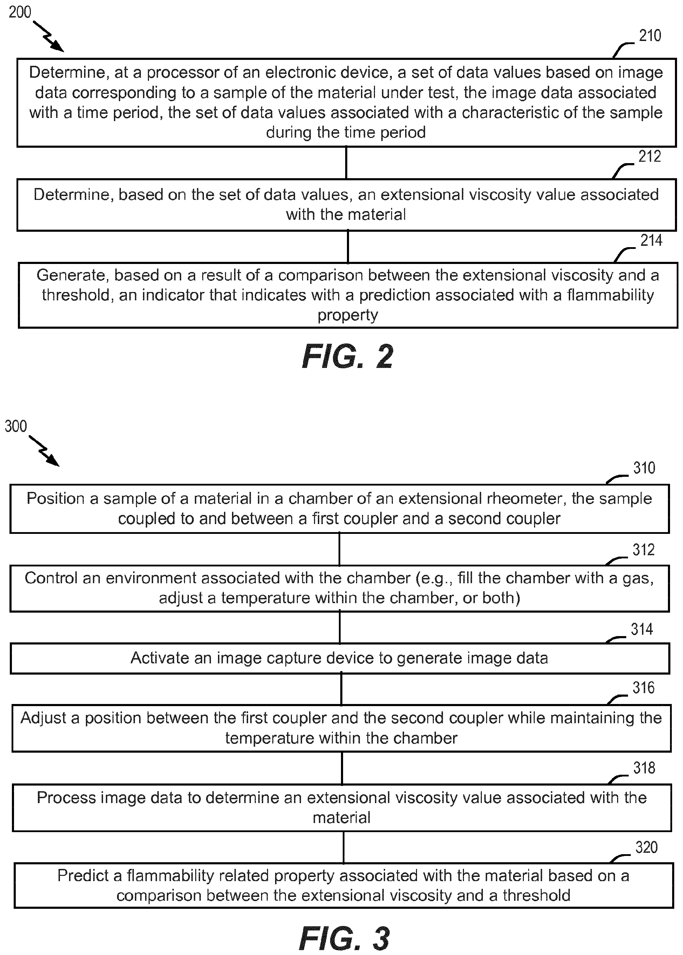

Memory 168 may be configured to store instructions 192, one or more thresholds 194, and one or more data sets 195. Instructions 192 may be configured to, when executed by the one or more processors 164, cause the processor(s) 164 to perform operations as described further here. For example, the one or more processors 164 may perform operations as described with reference to FIGS. 2-3.

The one or more I/O devices 170 may include a mouse, a keyboard, a display device, the camera, other I/O devices, or a combination thereof. In some implementations, I/O device 170 may include image capture device 156.

Electronic device 118 may include or correspond a communications device, a mobile phone, a cellular phone, a satellite phone, a computer, a tablet, a portable computer, a display device, a media player, or a desktop computer. Additionally, or alternatively, the electronic device 118 may include a set top box, an entertainment unit, a personal digital assistant (PDA), a monitor, a computer monitor, a television, a tuner, a video player, any other device that includes a processor or that stores or retrieves data or computer instructions, or a combination thereof.

During operation of system 100, a test of sample 119 may be performed. To illustrate, sample 119 of a material is attached to couplers 134, 136. Sample 119 may include a cylindrical sample melt that was cast using a hot press under vacuum was placed. Sample 119 may have an initial radius of $R_0$ = 2.25 mm and couplers 134, 136, may be spaced apart (e.g., an initial starting gap) of $L_0$ = 2.25 mm. The material may include a polymer that includes one or more FR additives. In some implementations, the material includes a flame retardant polycarbonate resin.

After sample 119 is placed in chamber 122, a covering (e.g., a door or panel) of rheometer 114 is secured and heat element 146 is activated to heat a temperature within chamber 122 to a particular temperature. For example, temperature controller 172 may send one or more temperature control signals 173 to heat element 146 to raise the temperature within chamber 122. In some implementations, a temperature within chamber 122 is raised at a rate of 5 °C/min. In some implementations, the particular temperature may be maintained within chamber 122 for a time period to enable sample 119 to reach an equilibrium temperature.

Additionally, after sample 119 is placed in chamber 122, gas supply 150 is activated to provide gas 152 to chamber 122. For example, environment controller 174 may send one or more environment control signals 175 to gas supply 150.

After the particular temperature within chamber 122 is reached, motor controller 178 may send one or more motion control signals 179 to motor 120 to initiate movement of first coupler 134 with respect to second coupler 136 to stretch sample 119. For example, motor 120 may be initiated to move couplers 134, 136 to be separated by a particular distance. In some implementations, motor 120 may move first coupler 134 at a velocity of 0.01 m/s$^2$ until a particular

distance (e.g., a separation) of $3L_0 = 6.75$ mm is reached.

In association with (e.g., before, during, and/or after) movement of first coupler 134, image capture device 156 may generate image data 185. For example, image capture device 156 may capture a diameter of sample 119 (e.g.,. a filament) for subsequent image processing and/or measurement. A measurement of the diameter may be constrained by a maximum magnification of an lens and/or the pixalation of an image capture sensor. To illustrate, in an illustrative, non-limiting example, the diameter measurements may have a resolution of 6.3 $\mu$m/pixel based on the maximum magnification of the lens and the pixalation of the camera sensor.

Electronic device 118 (e.g., processor 164) may process the image data 185 to measure one or more characteristics (e.g., a diameter) of the stretched sample. To illustrate, during and/or after movement of couplers 134, 136 is (e.g., after a stretch of sample 119), the capillary thinning of the liquid bridge (of sample 119) formed between couplers 134, 136 produces a uniaxial extensional flow from which the extensional viscosity and/or relaxation time of the test fluid can be measured. The time diameter data can then be in turn be used to measure the extensional viscosity of the material of sample 119 (e.g., a polymer melt) using the equation:

$$\eta_E = \frac{\sigma}{dD_{mid} / dt}$$

where, $\eta_E$ is the extensional viscosity [Pa.s], $\sigma$ is the surface tension [N/m], $D_{mid}$ is the diameter decay of the sample [m] and t is the time interval between each frame or diameter data point [s]. In some implementations, diameter measurements corresponds to the decay in the diameter after the stretch is given to a sample (e.g., 119).

Processor (e.g., 164, 180) may include an edge detection software (e.g., 184), such as Edgehog, to capture the diameter decay in time with subpixel resolution. Diameter values corresponding to sample 119 may be stored in memory 168 as first data set 196. In some implementations, to calculate the extensional viscosity (of a material of the sample), predictor 182 (e.g., viscosity calculator 186) may fit the diameter decay with a spline (e.g., spline interpolation is performed) and then differentiated. To further illustrate, to calculate an apparent or estimated extensional viscosity, diameter measurements as a function of time are fit with a spline to smooth the data and then differentiated.

Based on the extensional viscosity for the particular temperature, predictor 182 (e.g., comparator 188) may generate a prediction associated with a flammability related property of the material (e.g., sample 119). For example, predictor 182 (e.g., comparator 188) may compare the extensional viscosity to one or more thresholds 194. For example, the one or more thresholds 194 may include a first threshold associated with a value of 35,000 pascal seconds (Pa.s), a second threshold associated with a value of 10,000 pascal seconds (Pa.s), and/or one or more other thresholds.

Based on the comparator 188, predictor 182 (e.g., comparator 188) may generate a prediction associated with a flammability related property of the material (e.g., sample 119) and generate a notification 190 that indicates the prediction. Notification 190 may be provided (e.g., sent) via the one or more interfaces 160 and/or the one or more I/O devices 170. In some implementations, the prediction may be associated with a flammability rating, such as a UL 94 Flammability Standard. To illustrate, based on an average extensional viscosity being greater than or equal to a first threshold associated with a value of 35,000 pascal seconds (Pa.s) within a range of 180-200 degrees Celsius (°C) above the glass transition temperature ($T_g$) of the material, the material may be predicted to have achieve a UL 94 V0 rating. As another example, based on an average extensional viscosity being greater than or equal to a value of 10,000 pascal seconds (Pa.s) and/or less than or equal to a value of 35,000 pascal seconds (Pa.s) within a range of 180-200 degrees Celsius (°C) above the glass transition temperature ($T_g$) of the material, the material may be predicted to have achieve a UL 94 V2 rating.

In some implementations of system 100, after sample 119 is tested at a first temperature, a second sample of the material may be tested. For example, the second sample may be positioned in chamber 122 and chamber 122 may be heated to a second temperature. While the chamber 122 is at the second temperature, a stretch operation may be performed on the second sample and image capture device 156 may generate corresponding second image data. The second image data may be processed to determine diameter values correspond to the second sample that are stored in memory 168 as second data set 198. An extensional viscosity for the second sample (e.g., second data set 198) may be determined and combined with the extensional viscosity value for sample 119 (e.g., first data set 196). For example, the two extensional viscosity values may be averaged and the average may be compared to the one or more thresholds 194 to determine the prediction associated with the material.

In some implementations, sample 119 may be heated to a temperature above a glass transition temperature (Tg) of the material and/or a polymer (e.g., polycarbonate) included in the material. For example, sample 119 may be heated to a temperature within a range that has been correlated to a viscosity value that indicates reduction or prevention of drips associated with an FR additive. To illustrate, the range (e.g., an inclusive or exclusive range) of 180-200 degrees Celsius (°C) above $T_g$ may be used.

In a particular implementations, processor 164 (e.g., 182) is coupled to memory 168 and configured to execute instructions 192 to cause processor 164 (e.g., 182) to perform operations including determining a set of data values (e.g., 196) based on image data 185 corresponding to a test of sample 119 of a material performed at extensional rheometer 110. The image data may be associated with a time period and a temperature. The set of data values (e.g., 196) may be associated with a characteristic (e.g., a diameter) of sample 119 during the time period. The operations may further include determining, based on the set of data values (e.g., 196), an extensional viscosity value associated with the material, and generating, based on a result of a comparison between the extensional viscosity and a threshold (e.g., 194), notification 190 that indicates the prediction associated with the flammability related property of the material.

Thus, system 100 describes techniques to predict a flammability related property (e.g., an anti-drip property) of a material. For example, system 100 may provide predictions that are particularly robust based on testing of samples having an initial diameter of 1.5 mm-3 mm. System 100 may use extensional rheometer 114 to advantageously mimic the process of dripping, similar to how a polymer would form a drop and drip during an actual fire, and may use electronic device 118 to generate an extensional viscosity value at one or more temperatures. To illustrate, the extensional viscosity may be correlated to temperatures relevant to flame drips, thereby enabling predictions of a flammability related property (e.g., an anti-drip property) of a material, which have been unavailable using conventional techniques, such as Thermal Gravimetric Analysis, Mass Cone calorimetry and Pyrolysis-combustion flow calorimeter, that investigate the influence of FR additives. Accordingly, system 100 enables an effect of a particular FR additive of a material to be determined/predicted as it relates to a flammability related property (e.g., a standard rating) of a material. The prediction of the flammability related property (e.g., the standard rating) may be determined independent of and/or without performance of the standard-based test, such as the UL 94 standard test. Accordingly, system 100 enables a material formulation (e.g., an FR formulation of a polymer and one or more FR additives) to be optimized for a target or desired FR rating, such as a particular UL 94 rating. Additionally, system 100 enables and promotes development of new materials, such as materials that include an ultra-low halogen additive or a halogen free additive (e.g., KSS salt), and other ultra-low halogen or a halogen free crosslinkable materials, with flammability properties for a target or desired FR rating. Such optimization and/or development may be achieved with greater precision, in a shorter amount of time, at a reduced cost as compared to conventional techniques and/or testing to determine the flammability related property (e.g., the standard rating) of a material.

Referring to FIGS. 2 and 3, methods of generating a prediction associated with a flammability related property of a material are shown. In some implementation, the material may include an ultra-low halogen additive or a halogen additive. Each of the methods of FIGS. 2 and 3 may be performed or executed by one or more devices, such as one or more devices of system 100. To illustrate, each of the methods of FIGS. 2 and 3 may be performed by extensional rheometer 114, electronic device 118 (e.g., processor 164, 180, predictor 182, and/or memory 168), or a combination thereof.

Referring to FIG. 2, a method 200 for generating a prediction associated with a flammability related property of a material is shown. Method 200 may include determining, at a processor of an electronic device, a first set of data values based on first image data corresponding to a first sample of the material under test, at 210. The electronic device may include or correspond to electronic device 118. The first image data, such as image data 185, may be associated with a first time period. The first set of data values, such as first data set 196, may be associated with a characteristic of the first sample during the first time period. In a particular implementation, the characteristic is associated with a diameter of the first sample.

Method 200 also includes determining, based on the first set of data values, an extensional viscosity value associated with the material, at 212. For example, the extensional viscosity value may be determined by the processor 164 (e.g., the predictor 182). Method 200 also includes generating, based on a result of a comparison between the extensional viscosity and a first threshold, a notification (e.g., 190) that indicates the prediction associated with the flammability related property of the material, at 214. To illustrate, the first threshold may include or correspond to the one or more thresholds 194. The prediction may indicate the flammability rating is associated with a UL 94 Flammability Standard, an anti-drip property in the material, or both.

In some implementations, method 200 may include, after the first sample is positioned between a first coupled and a second coupler within a chamber (e.g., 122) of a extensional rheometer (e.g., 114), maintaining the first temperature within the chamber, and initiating movement of the first coupler and the second coupler. The first temperature is greater than a glass transition temperature (Tg) of the material (or a polymer of the material). An initial diameter of the first sample under test may be greater than or equal to 1.5 mm and/or less than or equal to 3 mm. Additionally, or alternatively, method 200 may also include providing a gas (e.g., 152), such as nitrogen, into the chamber; receiving the image data from a camera, or both.

In some implementations, method 200 may include determining the first set of data values based on the first image data includes performing edge detection on a first image based on the first image data, and determining a first data value of the first set of data values. The first image may correspond to a first time during the first time period, the first data value may be associated with a first diameter of the first sample at the first time. Determining the first set of data values may also include performing edge detection on a second image based on the first image data, and determining a second data value of the first set of data values. The second image may correspond to a second time during the first time period, and the second data value may be associated with a second diameter of the first sample at the second time. In some such implementations, determining the extensional viscosity value includes performing spline interpolation on the first set of data to generate a first interpolated data set, and performing a differential operation on the first interpolated data set.

In some implementations, method 200 may include, prior to performing the comparison between the extensional viscosity and the first threshold, performing a comparison between the extensional viscosity and a second threshold, and determining that the extensional viscosity is less than or equal to the second threshold. In some implementations, the first threshold or the second threshold corresponds to a value of 35,000 pascal seconds (Pa.s). Additionally, or alternatively, each of the first temperature and the second temperature may be greater than a glass transition temperature (Tg) of the material (or a polymer of the material). In some such implementations, the first temperature is different from the second temperature. In a particular implementation each of the first temperature and the second temperature is within a range of 180-200 degrees Celsius (°C) above the glass transition temperature (Tg) of the material (or a polymer of the material).

In some implementations, method 200 may include determining, at the processor, a second set of data values (e.g., 198) based on second image data corresponding to a second sample of the material. The second image data may be associated with a second time period and the second set of data values may be associated with a characteristic of the second sample during the second time period. In some such implementations, the extensional viscosity value is further determined based on the second set of data values.

In some implementations, the material may include 100 grade PCP in a weight percentage of 18.3-18.9 and KSS in a weight percentage of 0.02-0.55. Additionally, or alternatively, the material may include potassium perfluorobutane sulfonate in a weight percentage of 0.03-0.05. Particular examples of the material include: 100 grade PCP = 18.849 wt% and KSS = 0.03 wt%; 100 grade PCP = 18.779 wt% and KSS = 0.08 wt%; 100 grade PCP = 18.579 wt% and KSS = 0.3 wt%; 100 grade PCP = 18.379 wt% and KSS = 0.5 wt%; or 100 grade PCP = 18.779 wt% and KSS = 0.04 wt%. In other implementations, the material may include PCP 1300 in a weight percentage of 57.6-58.2 and KSS in a weight percentage of 0.02-0.55. Additionally, or alternatively, the material may include potassium perfluorobutane sulfonate in a weight percentage of 0.03-0.05. Particular examples of the material include: PCP 1300 = 58.119 wt% and KSS = 0.03 wt%; PCP 1300 = 58.069 wt% and KSS = 0.08 wt%; PCP 1300 = 57.849 wt% and KSS = 0.3 wt%; PCP 1300 = 57.649 wt% and KSS = 0.5 wt%; or PCP 1300 = 58.069 wt% and KSS = 0.04 wt%. In another particular example of the material includes PCP 1300 = 58.069 wt%, KSS = 0.04 wt%, and Potassium Perfluorobutane Sulfonate = 0.04 wt%.

Referring to FIG. 3, a method 300 for generating a prediction associated with a flammability related property of a material is shown. Method 300 includes positioning a sample of a material in a chamber of an extensional rheometer, at 310. To illustrate, with reference to FIG. 1, sample 119 may be positioned in chamber 122 of extensional rheometer 114. The sample may be positioned such that it is coupled to and between a first coupler (e.g., 134) and a second coupler (e.g., 136) of the extensional rheometer. In some implementations, an initial diameter of the sample under test is greater than or equal to 1.5 mm and/or less than or equal to 3 mm. Method 300 may also include controlling an environment associated with the chamber, at 312. Controlling the environment may include controlling a temperature, air quality, pressure, a combination thereof, or the like.

The method 300 may further include activating an image capture device to generate image data, at 314. To illustrate, with reference to FIG. 1, image capture device 156 may be activated to generate image data 185. The method 300 may also include adjusting a position between the first coupler and the second coupler while maintaining the temperature within the chamber, at 316. For example, motor 120 may be activated to adjust a spacing between couplers 134, 136 to stretch sample 119.

The method 300 may include processing image data to determine to an extensional viscosity value associated with the material, at 318. For example, referring to FIG. 1, processor (e.g., image processor 180, predictor 182, and/or viscosity calculator 186) may be configured to determine the extensional viscosity value based at least in part on image data 185. The method 300 further includes predicting a flammability related property associated with the material based on a comparison between the extensional viscosity and a threshold, at 320. For example, a prediction of the flammability related property may be indicated by or included in notification 190.

Thus, each of method 200 and method 300 describes techniques to predict a flammability related property (e.g., an anti-drip property) of a material. The techniques described with reference to method 200 and method 300 may be based on or utilize an extensional rheometer (e.g., 114) to advantageously mimic the process of dripping, similar to how a polymer would form a drop and drip during an actual fire. Additionally, or alternatively, the techniques described with reference to method 200 and method 300 may utilize an electronic device (e.g., 118) to generate an extensional viscosity value at one or more temperatures. Accordingly, each of method 200 and method 300 enables an effect of a particular FR additive of a material to be determined/predicted as it relates to a flammability related property (e.g., a standard rating) of a material. The prediction of the flammability related property (e.g., the standard rating) may be determined independent of and/or without performance of the standard-based test, such as the UL 94 standard test.

Accordingly, each of method 200 and method 300 enables a material formulation (e.g., an FR formulation of a polymer and one or more FR additives) to be optimized for a target or desired FR rating, such as a particular UL 94 rating. Additionally, each of method 200 and method 300 enables and promotes development of new materials, such as materials that include an ultra-low a halogen additive or a halogen free additive (e.g., KSS salt), with flammability properties for a target or desired FR rating. Such optimization and/or development may be achieved with greater precision, in a shorter amount of time, at a reduced cost as compared to conventional techniques and/or testing to determine the flammability related property (e.g., the standard rating) of a material.

The operations described with reference to the methods of FIGS. 2-3 may be controlled by one or more a processing unit such as a central processing units (CPUs), controllers, field-programmable gate array (FPGA) devices, application-specific integrated circuits (ASICs), other hardware devices, firmware devices, or any combination thereof. It is noted that one or more operations described with reference to one of the methods of FIGS. 2-3 may be combined with one or more operations of another of FIGS. 2-3. For example, one or more operations of method 200 may be combined with one or more operations of method 300.

## EXPERIMENTAL RESULTS

Tests on one or more materials were performed using an extensional rheometer, such as extensional rheometer 114 as described with reference to FIG. 1. The extensional rheometer (e.g., a capillary breakup extensional rheometer (CaBER)) used in the tests was configured for high temperature measurements within the chamber (e.g., 122) of up to 400 °C using heat elements (146) including three 250 Watt (W) resistance heaters (Omega-WS series). A temperature within the chamber was configured to be controlled using a PID temperature controller (Omega-CN-2110) with a temperature accuracy of +/- 2 °C.

The extensional rheometer included an inner and outer box (e.g., housing 121) fabricated from steel plates with insulation (e.g., 128) between the two boxes to reduce heat transfer out of the chamber. The thickness of the insulation was designed to maintain an inside temperature of T = 400 °C without exceeding an outside surface 124 temperature of 35 °C. The insulation included a silica-based insulator (Microsil, Zircar) having a thickness of 6.25 cm with a thermal conductivity of k =0.024 W/mK.

Rectangular openings (e.g., windows 130) were cut into the sides of both the inner and outer box and covered with a Pyrex glass to allow optical access for a camera sensor (e.g., 156) to have allow for visualization of a diameter of a sample (e.g., 119) to be measured optically as a function of time. The diameter measurements had a resolution of 6.3 $\mu$m/pixel based on a maximum magnification of the lens and the pixalation of the camera sensor. Additionally, the extensional rheometer was configured to be receive a supply of nitrogen and air into the chamber so that measurements could be made in an inert or an oxygen rich environment.

A top plate (e.g., 134) was connected to a liner motor (LinMot-C1250) (e.g., 120) using a glass mica rod (e.g., 138) to reduce heat flow out of the chamber and minimize the risk of heat damages to the motor. The motor was capable of stretching the sample (e.g., the fluid) at speeds up to 200 mm/s.

An electronic device (e.g., 118), such as a computer, was installed with an edge detection software (Edgehog) that was used to capture the diameter decay with time with subpixel resolution. In order to calculate the extensional viscosity, the diameter decay was fit with a spline and then differentiated.

In tests corresponding to the experiments described herein, cylindrical sample melts (e.g., 119) were cast using a hot press under vacuum. Each of the samples had an initial radius of approximately $R_0$ = 2.25 mm. Each of the samples was placed between two cylindrical plates (e.g., 134, 136) at room. The plates had an initial starting gap of $L_0$ = 2.25 mm. The extensional rheometer was then turned on and heated to the desired experimental temperature with a ramp of 35 °C/min. Once the chamber reached the desired temperature, the sample was allowed to reach equilibrium by remaining at the temperature for a minute before beginning of the stretch. The sample was then stretched with the top plate (e.g., 134) moving at a velocity of 0.01 m/s$^2$ until a particular distance (e.g., a separation between plates) of $3L_0$ = 6.75 mm was reached. The stretch was then stopped and the capillary thinning of the liquid bridge that was formed between the two endplates subsequently produced a uniaxial extensional flow from which the extensional viscosity and relaxation time of the test fluid could be measured and used to determine (e.g., estimate) the extensional viscosity of these polymer melts. The extensional viscosity was calculated directly from the measurement of diameter decay. To illustrate, to calculate the apparent extensional viscosity, the diameter measurements as a function of time were first fit with a spline to smooth the data and then differentiated. Based on the extensional viscosity measured in the temperature range (e.g., the range from Tg + 180 °C to Tg + 220 °C of the material or a polymer included in the material), the probability of passing of a polycarbonate mixed with various flame retardant salts was predicted.

## I First Experiment

Hyper-branched polycarbonates with HBN (Hydroxy Benzo Nitrile, commercial CFR resin) end group have strong UL94 V0 performance with Rimar salt as an additive. Unfortunately, the impact properties of these resins were poor when compared to a standard polycarbonate resin. Accordingly, the first experiment was performed to develop a formulae with halogen free additive (KSS salt) with improved impact properties. In other words, the objection of the first experiment was to design commercial CFR formulations with a halogen free additive for better impact properties while maintaining flame robustness. The developed formulations are presented below in TABLE 1 and TABLE 2. TABLE 1 includes formulations of commercial LUX 7630 C for ultra-low halogen or halogen free additives. TABLE 2 includes formulations of commercial CFR9111 ultra-low halogen or halogen free additives.

**TABLE 1**

| Item Description | Unit | Batch 2, 0.03 KSS, No Rimar | Batch 3, 0.08 KSS, No Rimar | Batch 4, 0.3 KSS, No Rimar | Batch 5, 0.5 KSS, No Rimar | Batch 6, 0.04 KSS + 0.04 Rimar |
|---|---|---|---|---|---|---|
| POTASSIUM PERFLUOROBUTANE SULFONATE (Global Master) | % | | | | | 0.04 |
| KSS (Global Master) | % | 0.03 | 0.08 | 0.3 | 0.5 | 0.04 |
| PENTAERYTHRITOL TETRASTEARATE | % | 0.27 | 0.27 | 0.27 | 0.27 | 0.27 |
| OCTAPHENYLCYCLOTETRASIL OXANE | % | 0.1 | 0.1 | 0.1 | 0.1 | 0.1 |
| DOVERPHOS S-9228 PHOSPHITE ANTIOXIDANT | % | 0.017 | 0.017 | 0.017 | 0.017 | 0.017 |
| HINDERED PHENOL ANTIOXIDANT | % | 0.05 | 0.05 | 0.05 | 0.05 | 0.05 |
| Polymethylphenyl siloxane | % | 0.4 | 0.4 | 0.4 | 0.4 | 0.4 |
| PHOSPHITE STABILIZER | % | 0.014 | 0.014 | 0.014 | 0.014 | 0.014 |
| 2-(2'HYDROXY-5-T-OCTYLPHENYL)-BENZOTRIAZOLE | % | 0.27 | 0.27 | 0.27 | 0.27 | 0.27 |
| PCP 1300 | % | 2 | 2 | 2 | 2 | 2 |
| THPE Branched PC Resin (ML6739-111N) | % | 28 | 28 | 28 | 28 | 28 |
| Branched THPE, HBN Endcapped PC | % | 50 | 50 | 50 | 50 | 50 |
| 100 GRADE PCP | % | 18.849 | 18.799 | 18.579 | 18.379 | 18.799 |
| High Molecular Weight OQ | % | | | | | |

**TABLE 2**

| Item Description | Unit | Batch 2, 0.03 KSS, No Rimar in CFR9111 | Batch 3, 0.08 KSS, No Rimar in CFR9111 | Batch 4, 0.3 KSS, No Rimar in CFR9111 | Batch 5, 0.5 KSS, No Rimar in CFR9111 | Batch 6, 0.04 KSS + 0.04 Rimar in CFR9111 |
|---|---|---|---|---|---|---|
| POTASSIUM PERFLUOROBUTANE SULFONATE (Global Master) | % | | | | | 0.04 |
| KSS (Global Master) | % | 0.03 | 0.08 | 0.3 | 0.5 | 0.04 |
| PENTAERYTHRITOL TETRASTEARATE | % | 0.27 | 0.27 | 0.27 | 0.27 | 0.27 |

(continued)

| Item Description | Unit | Batch 2, 0.03 KSS, No Rimar in CFR9111 | Batch 3, 0.08 KSS, No Rimar in CFR9111 | Batch 4, 0.3 KSS, No Rimar in CFR9111 | Batch 5, 0.5 KSS, No Rimar in CFR9111 | Batch 6, 0.04 KSS + 0.04 Rimar in CFR9111 |
|---|---|---|---|---|---|---|
| OCTAPHENYLCYCLOTETRASILOXANE | % | 0.1 | 0.1 | 0.1 | 0.1 | 0.1 |
| DOVERPHOS S-9228 PHOSPHITE ANTIOXIDANT | % | 0.017 | 0.017 | 0.017 | 0.017 | 0.017 |
| HINDERED PHENOL ANTIOXIDANT | % | 0.05 | 0.05 | 0.05 | 0.05 | 0.05 |
| Polymethylphenyl siloxane | % | 0.4 | 0.4 | 0.4 | 0.4 | 0.4 |
| PHOSPHITE STABILIZER | % | 0.014 | 0.014 | 0.014 | 0.014 | 0.014 |
| PCP 1300 | % | 58.119 | 58.069 | 57.849 | 57.649 | 58.069 |
| THPE Branched PC Resin (ML6739-111N) | % | 11 | 11 | 11 | 11 | 11 |
| Branched THPE, HBN Endcapped PC | % | 20 | 20 | 20 | 20 | 20 |
| 100 GRADE PCP | % | | | | | |
| 2-(2'HYDROXY-5-T-OCTYLPHENYL)-BENZOTRIAZOLE | % | | | | | |
| High Molecular Weight OQ | % | 10 | 10 | 10 | 10 | 10 |

The developed formulations of TABLE 1 and TABLE 2 were tested using the extensional rheometer. Referring to the formulations of TABLE 1, the measurements demonstrated threshold viscosities of 35,000 Pa.s and above in the range from Tg + 180 °C to Tg + 220 °C for LUX7630 (Melt flow index of 4) formulations. Stated in another manner, the measurements for the formulation of TABLE 1 predicted a V0 rating (no inflamed drips) before molding test samples for UL 94 testing. UL 94 testing was subsequently performed on samples of the formulations of TABLE 1 that were configured for (e.g., defined by) UL 94 testing. TABLE 3 includes UL-94 vertical burn test rating for a flame bar thickness of 2 mm: LUX 7630 C Formulation. As shown in TABLE 3, all the materials showed a V0 rating in UL 94 testing.

**TABLE 3**

| Formulation Based on LUX 7630 C | Thickness 2 mm |
|---|---|
| Batch 2 -0.03 KSS, No Rimar | V0 |
| Batch 3- 0.08 KSS, No Rimar | V0 |
| Batch 4- 0.3 KSS, No Rimar | V0 |
| Batch - 0.5 KSS, No Rimar | V0 |
| Batch 6- (0.04 KSS+ 0.04 Rimar) | V0 |

Referring to the formulations of TABLE 2, the tested materials for CFR9111 (Melt flow index of 4) showed a extensional viscosity of about 10,000 Pa.s in the range from Tg + 180 °C to Tg + 220 °C, which is less than the threshold level (e.g., 35,000 Pa.s). This indicated that these formulations did not build enough viscosity to reduce or arrest the drips and, thus, the formulations received V2 rating in UL 94 testing. For example, UL 94 testing was subsequently performed on samples of the formulations of TABLE 2 that were configured for (e.g., defined by) UL 94 testing. TABLE 4 includes UL-94 vertical burn test rating for a flame bar thickness of 2 mm: LUX 7630 C Formulations. As shown in TABLE 4, all the materials showed a V2 rating in UL 94 testing.

**TABLE 4**

| Formulation Based on CFR9111 | Thickness-2 mm |
|---|---|
| Batch 2 -0.03 KSS, No Rimar | V2 |
| Batch 3- 0.08 KSS, No Rimar | V2 |
| Batch 4- 0.3 KSS, No Rimar | V2 |
| Batch - 0.5 KSS, No Rimar | V2 |
| Batch 6- (0.04 KSS+ 0.04 Rimar) | V2 |

## II. Second Experiment

In a second experiment, polycarbonate mixed with various flame retardant salts (FR) were supplied by SABIC. Before being tested in shear and extensional rheometers, the pellets were first molded into samples to fit the plate geometry (of the extensional rheometer) in a hot press under vacuum at T = 250 °C.

The various composition of the flame retardant salts mixed with the PC are listed below:

a) Linear PC

b) 0.08wt % Rimar Salt (Potassium Perfluorobutane Sulfonate) and PC- (PC/Rimar)

c) PC and 0.3wt% KSS (Potassium Diphenyl Sulfone-3-Sulfonate)-(PC/KSS)

d) PC and 0.5wt% TSAN (Polytetrafluoroethylene encapsulated with styrene acrylonitrile resin) -(PC/TSAN)

e) PC, 0.08wt% Rimar Salt, 0.1wt% T-SAN and 0.2 mol% 1.1.1.tris hydroxy phenyl ethan (PC/Rimar/TSAN/THPE)

f) PC, 0.3wt% KSS and 0.3wt% TSAN (PC/KSS/TSAN)

Referring to TABLE 5, a table of the various composition (e.g., compositions a-f identified above) of the flame retardant salts mixed with the PC and each composition's UL-94 vertical burn test rating for a flame bar thickness of 1.2 mm is shown.

**TABLE 5**

| SAMPLE | UL-94 rating |
|---|---|
| PC/Rimar (e.g., composition b) | V0 |
| PC/TSAN (e.g., composition d) | V0 |
| PC/KSS (e.g., composition c) | V2 |
| PC/Rimar/TSAN/THPE (e.g., composition e) | V0 |
| PC/KSS/TSAN (e.g., composition f) | V0 |
| Linear PC (e.g., composition a) | V2 |

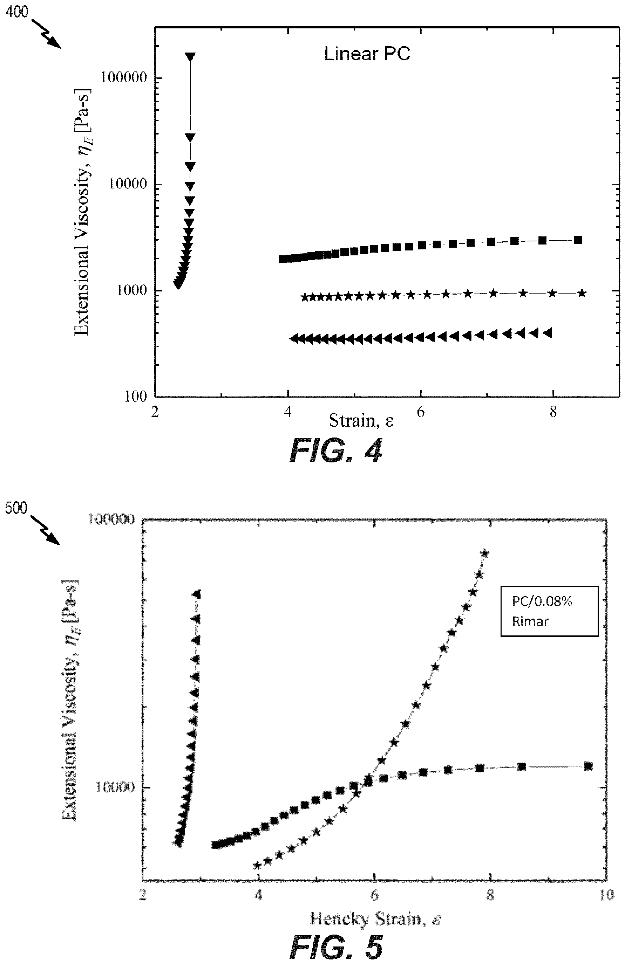

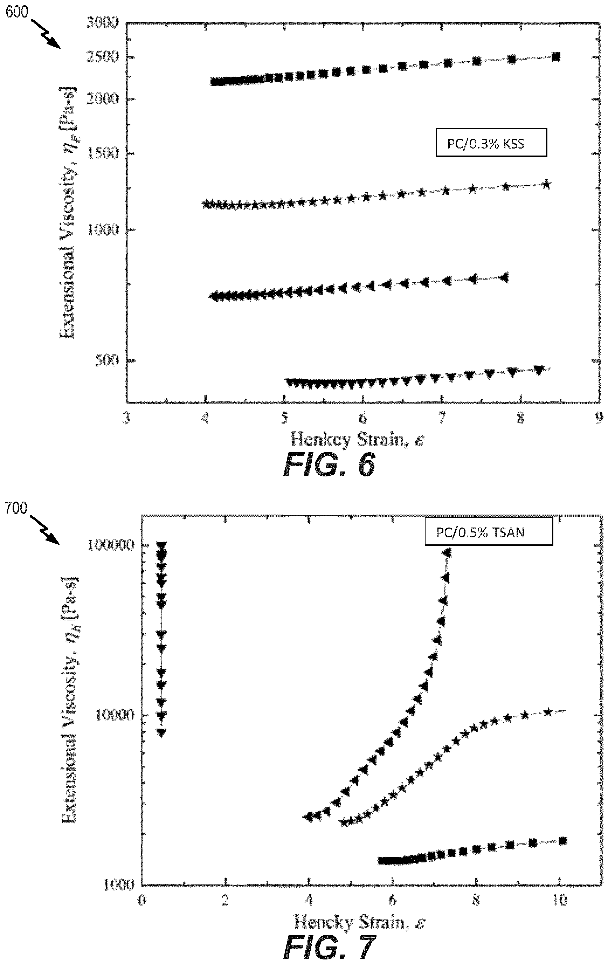

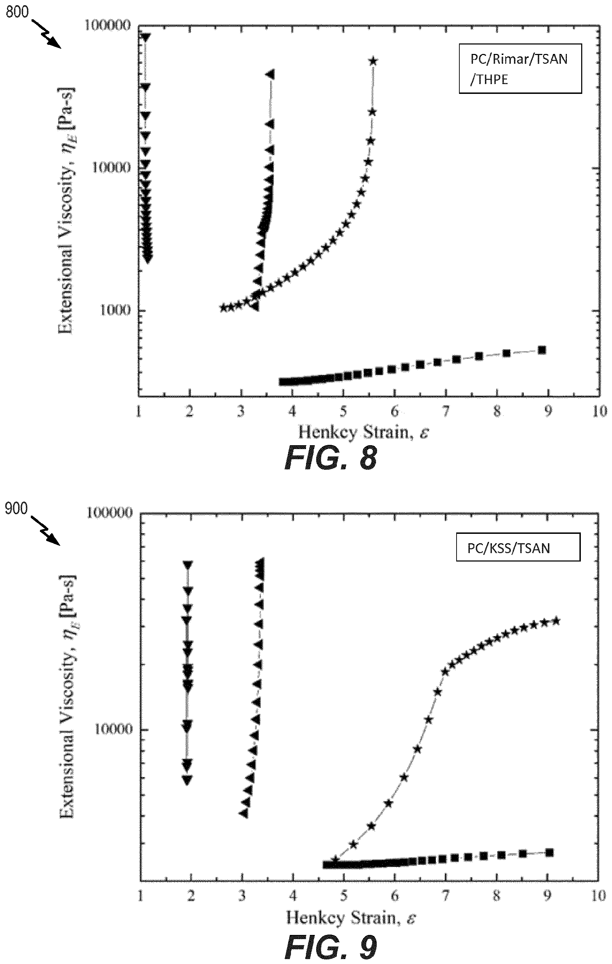

Referring to FIGS. 4-9, the extensional viscosities of samples (to fit the plate geometry of the extensional rheometer) of compositions a-f measured in the temperature range of T = 320 °C to T = 380 °C in the presence of nitrogen are shown. For example, FIG. 4 is associated with composition a (e.g., Linear PC); FIG. 5 is associated with composition b (e.g., PC/0.08% Rimar); FIG. 6 is associated with composition c (e.g., PC/0.3% KSS); FIG. 7 is associated with composition d (e.g., PC/0.55% TSAN); FIG. 8 is associated with composition e (e.g., PC/Rimar/TSAN/THPE); and FIG. 9 is associated with composition f (e.g., PC/KSS/TSAN). In each of FIGS. 4-9, capillary breakup extensional rheology measurement of extensional viscosity, , as a function of Hencky strain, , strain for the polycarbonate with various flame retardant salt in nitrogen at temperatures T = 320 °C (■),T = 340 °C (☆ ), T = 360 °C (◄) and T = 380 °C (▼).

Referring to FIGS. 4-9, all measurements are done in the presence of nitrogen. As shown in FIG. 4 for the linear PC, an initial temperature increase from T = 320 °C to T = 340 °C, the extensional viscosity decreased by a factor of two. On further increasing to T = 360 °C, a further decrease in the extensional viscosity is observed which is due chain scission happening at those temperatures. At temperature T =380 °C, a divergence in the extensional viscosity is observed which is due to significant crosslinking. The term divergence in the extensional viscosity may be defined as the point when the extensional viscosity rise becomes parallel to the y axis, because the diameter of the filament ceases to decay and the material behaves like a solid, which indicates an improvement in the anti-dripping characteristics of the polymer. A numerical value of the viscosity when the extensional viscosity diverges is defined to be 100000 Pa.s. The average of the steady state extensional viscosity can be calculated in the temperature range of T = 320°C to T = 360 °C. The steady state extensional viscosity is intended to mean the value of the extensional viscosity when it's not varying with strain or time. Thus, based on the calculation(s) as describe herein above, the average extensional viscosity of the linear PC from T = 320°C to T = 360 °C (which is in the range of [Tg + 180 °C to Tg + 200 °C]) is approximately (eta) linear=1500 Pa.s.

Referring to FIG. 5, similar trends was observed for the PC/0.08% Rimar where the divergence in extensional viscosity was observed at T = 340 °C. The average extensional viscosity for PC/0.08% Rimar was calculated to be (eta) PC/0.08% Rimar = 70000 Pa.s. It is noted that values for T = 380 °C have been omitted from FIG. 5 for ease of illustration since the extensional viscosity has already diverged at T = 360 °C.

Referring to FIGS. 6-9, similarly on calculating the average extensional viscosity for the other samples, it was found that for PC/0.3% KSS (eta) PC/0.3% KSS = 1583 Pa.s, for PC/0.5% TSAN it was 38,000 Pa.s, for PC/Rimar/TSAN/THPE it was 68,000 Pa.s, and for PC/0.3% KSS/0.3% TSAN it was 42,500 Pa.s.

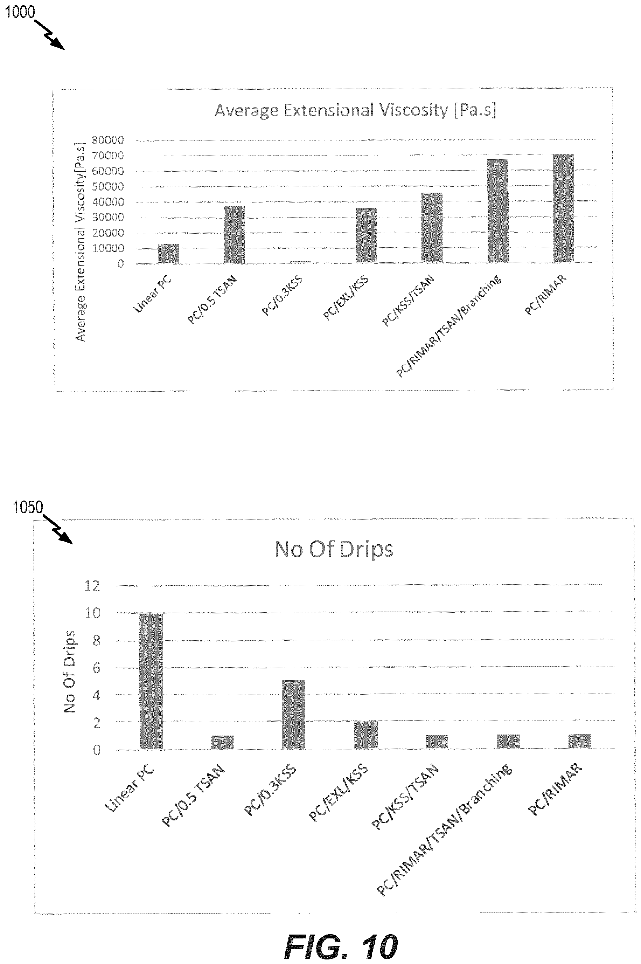

Referring to FIG. 10, graphs to show a correlation of Average Extensional Viscosity vs. Flame Drips For a Series of Representative Samples (e.g., to compositions a-f) are shown. For example, FIG. 10 shows a first graph 1000 and a second graph 1050. First graph 1000 shows a summary of average extensional viscosity (Pa.s) for compositions a-f. Second graph 1050 shows a number of flame drips for compositions a-f. FIG. 10 demonstrates the influence of extensional viscosity in reducing the drips. As shown in first and second graphs 1000, 1050, samples with better UL 94 ratings have the average extensional viscosity of 35,000 Pa.s or greater. In other words, an average extensional viscosity of 35,000 Pa.s and above (in the temperature range from Tg + 180 °C to Tg + 220 °C) indicates an avoidance of (or greatly reduced) drips in UL 94 testing.

The above specification and examples provide a complete description of the structure and use of illustrative embodiments. Although certain embodiments have been described above with a certain degree of particularity, or with reference to one or more individual embodiments, those skilled in the art could make numerous alterations to the disclosed embodiments without departing from the scope of this disclosure. As such, the various illustrative embodiments of the methods and systems are not intended to be limited to the particular forms disclosed. Rather, they include all modifications and alternatives falling within the scope of the claims, and embodiments other than the one shown may include some or all of the features of the depicted embodiments. For example, elements may be omitted or combined as a unitary structure, connections may be substituted, or both. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several embodiments. Accordingly, no single implementation described herein should be construed as limiting and implementations of the disclosure may be suitably combined without departing from the teachings of the disclosure.

The claims are not intended to include, and should not be interpreted to include, means-plus- or step-plus-function limitations, unless such a limitation is explicitly recited in a given claim using the phrase(s) "means for" or "step for," respectively.

**Claims**

1. A system comprising:

    an electronic device comprising a processor and a memory coupled to the processor, the memory storing instructions that, when executed by the processor, cause the processor to:

        determine a set of data values based on image data corresponding to a test of a sample of a material performed at an extensional rheometer, the image data associated with a time period and a temperature, the set of data values associated with a characteristic of the sample during the time period;
        determine, based on the set of data values, an extensional viscosity value associated with the material; and
        generate, based on a result of a comparison between the extensional viscosity and a threshold, a notification associated with a prediction of a flammability rating.

2. The system of claim 1, wherein:

the material comprises a flame retardant polycarbonate resin;
the threshold corresponds to a value of 35,000 pascal seconds (Pa.s);
the flammability rating is associated with a UL 94 Flammability Standard; or
the control device is further configured to:

receive the first image data from a camera; and
receive temperature data indicating the temperature value.

3. The system of claim 1, wherein:

the flammability rating is associated with a UL 94 Flammability Standard; and
the prediction indicates a UL 94 V0 rating is achievable for the material based on an average extensional viscosity value being greater than or equal to 35,000 pascal seconds (Pa.s) within a range of 180-200 degrees Celsius (°C) above the glass transition temperature ($T_g$) of the material; and/or
the prediction indicates a UL 94 V2 rating is achievable for the material based on an average extensional viscosity value being greater than or equal to 10,000 pascal seconds (Pa.s) within a range of 180-200 degrees Celsius (°C) above the glass transition temperature ($T_g$) of the material.

4. The system of any of claims 1 and 3, wherein:

the flammability rating is associated with a UL 94 Flammability Standard; and/or
the material includes an ultra-low halogen additive or a halogen additive crosslinkable material.

5. The system of any of claims 1-3, further comprising:

the extensional rheometer coupled to the electronic device, the extensional rheometer comprising:

a housing defining a chamber, the housing having a window incorporated therein;
a first coupler and a second coupler positioned within the chamber, the first and second coupler configured to hold a sample, the first coupler movable with respect to the second coupler; and
a heat element positioned within the chamber; and

an image capture device coupled to the electronic device and positioned to have a field of view through the window, the camera configured to generate the image data.

6. A method of generating a prediction associated with a flammability related property of a material, the method comprising:

determining, at a processor of an electronic device, a first set of data values based on first image data corresponding to a first sample of the material under test, the first image data associated with a first time period, the first set of data values associated with a characteristic of the first sample during the first time period;
determining, based on the first set of data values, an extensional viscosity value associated with the material; and
generating, based on a result of a comparison between the extensional viscosity and a first threshold, a notification that indicates the prediction associated with the flammability related property of the material.

7. The method of claim 6, wherein:

an initial diameter of the first sample under test is greater than or equal to 1.5 mm and/or less than or equal to 3 mm,
the prediction indicates the flammability rating is associated with a UL 94 Flammability Standard, an anti-drip property in the material, or both, and
optionally, the material comprises a flame retardant polycarbonate resin.

8. The method of any of claims 6-7, further comprising:

providing a gas into the chamber, wherein, optionally, the gas comprises nitrogen;
adjusting a temperature within the chamber to a first temperature; and
receiving the image data from a camera.

9. The method of any of claims 6-8, further comprising, after the first sample is positioned between a first coupled and a second coupler within a chamber of a extensional rheometer:

maintaining the first temperature within the chamber; and
initiating movement of the first coupler and the second coupler;
wherein, optionally, the first temperature is greater than a glass transition temperature (Tg) of the material.

10. The method of any of any of claims 6-9, wherein determining the first set of data values based on the first image data comprises:

performing edge detection on a first image based on the first image data, the first image corresponding to a first time during the first time period;
determining a first data value of the first set of data values, wherein the first data value is associated with a first diameter of the first sample at the first time;
performing edge detection on a second image based on the first image data, the second image corresponding to a second time during the first time period; and
determining a second data value of the first set of data values, wherein the second data value is associated with a second diameter of the first sample at the second time.

11. The method of any of claims 6-10, wherein determining the extensional viscosity value comprises:

performing spline interpolation on the first set of data to generate a first interpolated data set; and
performing a differential operation on the first interpolated data set.

12. The method of any of claims 6-11, further comprising:

prior to performing the comparison between the extensional viscosity and the first threshold, performing a comparison between the extensional viscosity and a second threshold;
determining that the extensional viscosity is less than or equal to the second threshold, wherein, optionally, the first threshold corresponds to a value of 35,000 pascal seconds (Pa.s).

13. The method of any of claims 6-12, wherein:

each of the first temperature and the second temperature is greater than a glass transition temperature (Tg) of the material, and wherein the first temperature is different from the second temperature; and
optionally, each of the first temperature and the second temperature is within a range of 180-200 degrees Celsius (°C) above the glass transition temperature (Tg) of the material.

14. The method of any of claims 6-14, further comprising:

determining, at the processor, a second set of data values based on second image data corresponding to a second sample of the material, the second image data associated with a second time period, the second set of data values associated with a characteristic of the second sample during the second time period,
wherein the extensional viscosity value is further determined based on the second set of data values, and
wherein, optionally, the material includes an ultra-low halogen additive or a halogen additive.

15. The method of any of claims 6-14, wherein the material comprises:

100 grade PCP in a weight percentage of 18.3-18.9 and KSS in a weight percentage of 0.02-0.55, and, optionally, potassium perfluorobutane sulfonate in a weight percentage of 0.03-0.05; or
PCP 1300 in a weight percentage of 57.6-58.2 and KSS in a weight percentage of 0.02-0.55, and, optionally, potassium perfluorobutane sulfonate in a weight percentage of 0.03-0.05.

FIG. 1

EP 3 605 058 A1

200

210

Determine, at a processor of an electronic device, a set of data values based on image data corresponding to a sample of the material under test, the image data associated with a time period, the set of data values associated with a characteristic of the sample during the time period

212

Determine, based on the set of data values, an extensional viscosity value associated with the material

214

Generate, based on a result of a comparison between the extensional viscosity and a threshold, an indicator that indicates with a prediction associated with a flammability property

## FIG. 2

300

310

Position a sample of a material in a chamber of an extensional rheometer, the sample coupled to and between a first coupler and a second coupler

312

Control an environment associated with the chamber (e.g., fill the chamber with a gas, adjust a temperature within the chamber, or both)

314

Activate an image capture device to generate image data

316

Adjust a position between the first coupler and the second coupler while maintaining the temperature within the chamber

318

Process image data to determine an extensional viscosity value associated with the material

320

Predict a flammability related property associated with the material based on a comparison between the extensional viscosity and a threshold

## FIG. 3

FIG. 4

FIG. 5

**FIG. 6**

**FIG. 7**

FIG. 8

FIG. 9

1000

1050

FIG. 10

Europäisches
Patentamt

European
Patent Office

Office européen
des brevets

# EUROPEAN SEARCH REPORT

Application Number

EP 18 18 6281

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2011 112362 A (SHIP PARTNERS LTD; KAWAJU TECHNOLOGY KK) 9 June 2011 (2011-06-09) | 1,2,4-15 | INV. G01N11/00 G01N25/50 |
| A | * Abstract; paragraphs [0066], [0078] - [0079], [0084] - [0085], [0094] - [0095]; figure 1 * | 3 | |
| Y | WO 02/14836 A2 (CAMBRIDGE POLYMER GROUP INC [US]) 21 February 2002 (2002-02-21) | 1,2,4-15 | |
| A | * Abstract; paragraphs [0032], [0039], [0005]; figure 1 * | 3 | |

TECHNICAL FIELDS
SEARCHED       (IPC)

G01N
C08L
C08K

The present search report has been drawn up for all claims

| Place of search | Date of completion of the search | Examiner |
|---|---|---|
| The Hague | 25 January 2019 | Kramer, Joanne |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 18 6281

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2019

| Patent document cited in search report | | Publication date | Patent family member(s) | | Publication date |
|---|---|---|---|---|---|
| JP 2011112362 | A | 09-06-2011 | JP | 5363954 B2 | 11-12-2013 |
| | | | JP | 2011112362 A | 09-06-2011 |
| WO 0214836 | A2 | 21-02-2002 | AU | 8647201 A | 25-02-2002 |
| | | | CA | 2419128 A1 | 21-02-2002 |
| | | | EP | 1322935 A2 | 02-07-2003 |
| | | | US | 2002116987 A1 | 29-08-2002 |
| | | | WO | 0214836 A2 | 21-02-2002 |

EPO FORM P0459

For more details about this annex : see Official Journal of the European Patent Office, No. 12/82